# EUROPEAN PATENT SPECIFICATION

(11) **EP 3 653 233 B1**
(45) Date of publication and mention of the grant of the patent: **17.08.2022**
(21) Application number: 19207224.7
(22) Date of filing: 05.11.2019
(51) Int. Cl.: A61L 27/24, A61L 27/36, A61L 27/38

(54) **DISPOSABLE MEDICAL DEVICE FOR MEDICATION OF PATIENT'S SKIN LESIONS AND RELATED PREPARATION AND USE METHODS**
MEDIZINISCHE EINWEGVORRICHTUNG ZUR MEDIKATION VON HAUTLÄSIONEN EINES PATIENTEN UND ZUGEHÖRIGES PRÄPARAT UND VERWENDUNGSVERFAHREN
DISPOSITIF MÉDICAL JETABLE DE TRAITEMENT DE LÉSIONS CUTANÉES DE PATIENTS ET PROCÉDÉS DE PRÉPARATION ET D'UTILISATION ASSOCIÉS

(30) Priority: 16.11.2018 IT 201800010390
(43) Date of publication of application: 20.05.2020
(73) Proprietor: Furlan, Diego, 20090 Segrate (MI) (IT)
(72) Inventor: Furlan, Diego, 20090 Segrate (MI) (IT)
(74) Representative: Mittler, Enrico

(56) References cited:
- EP-A1- 1 488 816
- EP-A1- 2 573 167
- EP-A1- 3 138 904
- WO-A1-2017/197154
- WO-A2-03/076604
- US-A- 6 039 760
- PHILLIPS T J: "BIOLOGIC SKIN SUBSTITUTES", JOURNAL OF DERMATOLOGIC SURGERY AND ONCOLOGY, NEW YORK, NY, US, vol. 19, 1993, pages 794-800, XP002929718,
- Thomas M Harris: "A Tissue Culture Perfusion Chamber with a Substratum of Reconstituted Collagen*", , 1966, XP055610127, Retrieved from the Internet: URL:https://pdfs.semanticscholar.org/5f7c/ f52ebea439fbd233297c14020c476c819550.pdf [retrieved on 2019-07-31]
- Anonymous: "VueLife Closed Culture System", , 2016, XP055610102, Retrieved from the Internet: URL:http://athica.com.br/wp-content/upload s/2016/05/FEP-Bags-Fittings-AFC.pdf [retrieved on 2019-07-31]

## Description

The present invention relates to a disposable medical device which is usable for medication of patient's skin lesions, such as wounds, burns, bedsores, chronic ulcers, diabetic foot and the like, and to a related use preparation method.

The healing of large chronic and acute wounds is a challenging task for operators in the sector: it requires several visits and frequent medication changes and often involves expensive therapeutic procedures. The primary objective is to close these wounds as quickly as possible. In a prepared wound bed, with a good granulation tissue and free of infection, the skin graft is the standard procedure to achieve this goal. Unfortunately, its application is often limited by the insufficient availability of autologous skin grafts. An alternative is the grafting of cultured keratinocytes and/or fibroblasts, possibly autologous. However, due to several complications and to the graft preparing process, which takes a long time, normally two to three weeks, to obtain a fragile and thin layer of cells of autologous cultured skin, the initial optimism for the autograft of autologous cells has gradually decreased.

An example of a technique currently used for the treatment of cutaneous wounds is described in the article "Biologicskin substitutes" by Tania J. Phillips published in the Journal of Dermatologic Surgery and Oncology, New York, NY, US, vol. 19, 1993, pages 794-800, XP002929718, figure 6, which is regarded as the closest prior art. There is disclosed a perfused bag for growing skin cells from a patient. Epithelial cells are injected into a sterile envelope containing a biodegradable mesh. The cells attach themselves to the mesh, multiply and begin to secrete collagen, i.e. proteins which help form connective tissues. Gradually, the cells and collagen proteins cover the mesh inside and outside to form a solid tissue. The replacement skin is sutured with the wound and the mesh gradually dissolves.

Another example is described in WO 03/076604 A2 and includes using a biocompatible cross-linked matrix, on which cultured epithelial cells are inoculated and cultured.

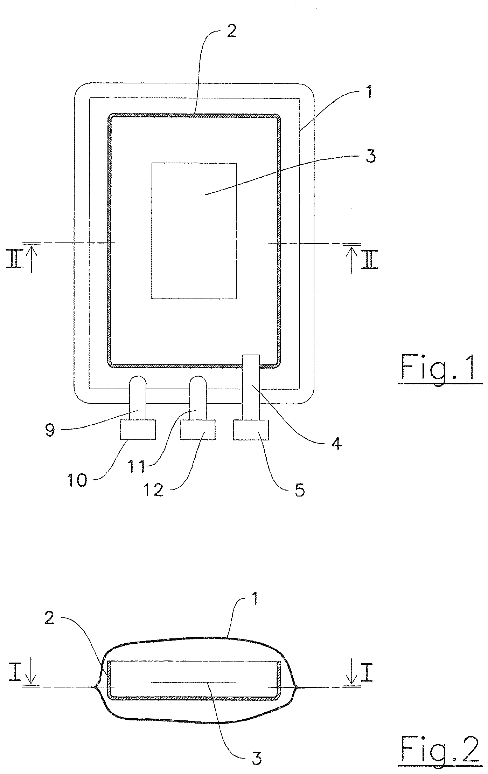

Yet another example is described in the article "A Tissue Culture Perfusion Chamber with a Substratum of Reconstituted Collagen" by Thomas M. Harris, 1996, XP055610127, figure 1, and includes using a screen disk covered with collagen placed inside a chamber which is then filled with a culture medium.

It is the object of the present invention to improve the prior art solutions, allowing a rapid and efficient availability of uniform flaps of engineered tissue to be transplanted onto the lesion of the patient.

To achieve this object, a disposable medical device has been devised according to the present invention as defined in claim 1.

The use of a collagen sheet, forming a perfectly uniform flat surface which is free from perforations, reliefs and/or depressions, is particularly suitable for the uniform growth of a skin replacement layer to be applied in an effectively curative manner and in a short time to the patient's wound.

This disposable device, with cell growth on the collagen started earlier within the device itself and then on the bed of the patient's wound, being always supported by the collagen sheet, results in a decisive shortening of the times during which the wound remains exposed with danger of contamination.

Since the cells are cultured on the collagen layer, there are no problems of fragility of the flap of epithelial tissue cultured in the laboratory and then applied to the patient.

Compared to transplantation from autologous skin, the amount of skin to be taken in the case of the device of the present invention is much lower.

A method for preparing use of the disposable medical device is defined in claim 2.

The constructional features of the device according to the invention will become apparent from the following detailed description of a possible embodiment thereof, shown by way of a non-limiting example in the accompanying drawings, in which:
figure 1 shows a top plan view of an example of the device according to the invention with an external envelope sectioned according to the line I-I in figure 2;
figure 2 shows a sectional view of the same device according to line II-II in figure 1;
figure 3 shows a plan view of a non-claimed variant of the device shown in the preceding figures.

The device diagrammatically shown in FIG. 1 and FIG. 2 comprises a sealed and sterilized external envelope 1, in the shape of a three-dimensional bag, inside which a tray or cup 2 made of plastic or glass is arranged, where a collagen sheet 3 is accommodated. The tray 2 is also intended to contain, in addition to the collagen sheet 3, a homogenate of skin cells and a culture medium.

The sterile envelope 1 is made of biocompatible plastic material such as EVA (ethylene-vinyl acetate) or PVC (polyvinyl chloride).

The tray 2 is made of biocompatible plastic materials such as PET-G (polyethylene terephthalate copolyester), PP (polypropylene), PE (polyethylene), polystyrene, nylon or glass.

The structure used as a substrate for the growth of epithelial cells, i.e. the collagen sheet 3, is a native type I collagen in the form of a spongy sheet or film obtained from bovine or equine flexor tendons with one of the known techniques such as that described in US patent 5,783,983. Collagen sterilization is achieved by gamma or beta irradiation. Collagen can be alone or added with glycosaminoglycans (GAG), preferably chondroitin sulfate.

From the tray 2 and through the corresponding lower part of the envelope or bag 1 a flexible tube 4 extends outwards, which ends with a sealed connector 5, through which it is possible to introduce a homogenized suspension of autologous epithelial cells into the chamber or cup 2, intended to adhere and grow on the surface of the collagen structure 3 to form a device which is ready for transplantation onto the patient's wound bed.

The connector 5 is of a type which is pierceable by a syringe needle or luer and capable of closing and self-sealing after extraction of the needle or luer at the end of the operation of introducing the cells into the chamber or tray 2. For example, but not necessarily, the connector 5 can be of the type described in EP 2 667 839 B1.

The cell homogenate introduced through the connector 5 consists of epithelial or dermal-epithelial cells taken from an area of intact skin of the patient, more precisely from the epidermis layer with possible involvement of the dermis, and subjected to shredding and homogenization by means of systems known *per se.* The cell homogenate is suspended in saline to facilitate the manipulation and subsequent syringe injection on the collagen sheet 3 through the connector 5 and the tube 4.

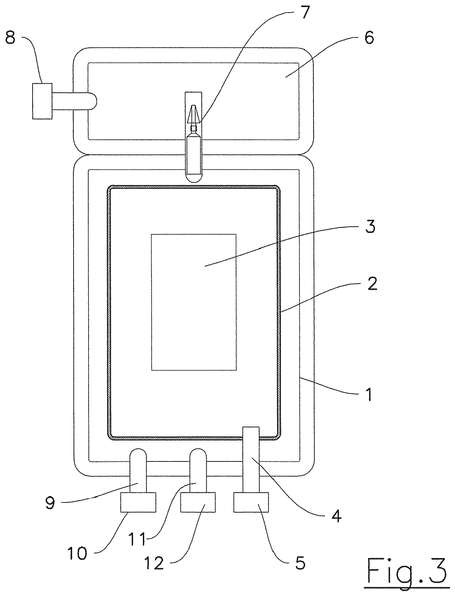

A saline is present inside the chamber or tray 2, in which the culture medium will be added. As an example not being part of the claimed invention, the culture medium can be taken from a leg 6 of the bag 1, to which it is connected by means of a fracture cone 7 and an introduction connector 8 (figure 3).

Next to the opening from which the tube 4 emerges there is another opening of the envelope 1 from which a tube 9 extends with a breakable closure cone (not shown), which ends with a sterile filter 10 (permeable to gas and water vapor, but impermeable to liquids) which allows the circulation of an atmosphere which is suitable for cell growth.

A further tube 11 with closure cap 12 extends from a further opening of the envelope 1 to allow the discharge of the exhausted culture medium after a time which can be evaluated over 48 hours.

The basic concept of the present invention is not to expand the initial tissue, but to seed the cells of the initial crushed, shredded and homogenized explant on a uniform flat collagen surface where the cell growth continues to confluence, i.e. until the cells, while growing, touch and no longer find empty spaces, partially on the culture medium for 48-96 hours and partially, until the end, directly on the wound bed.

Therefore, the present invention aims at continuing the proliferation and expansion of the dermal-epithelial cells obtained from the removal of the patient's skin (which can be only epidermis using a dermatome or epidermis and dermis using a scalpel) directly on the bed of the lesion after a short period (48-96 hours) of seeding and proliferation of the cells on the collagen substrate by means of specific culture media.

The procedure of using the device according to the present invention is as follows:
- a small aliquot of skin, of about 2 cm², is shredded and homogenized using one of the known techniques and suspended in a small amount of a sterile saline;
- the cell suspension is distributed on the surface of the collagen sheet 3 of the device through one of the openings of the sealed envelope 1, preferably by using a syringe without needle;
- a selected culture medium, which is specific for growing fibroblasts and/or keratinocytes, is loaded inside the sealed envelope;
- a bacteriostatic and bactericide solution is added if the operator is not sure on maintaining the sterile conditions during the collection, extraction, homogenization, suspension and injection procedure;
- the device is placed inside an incubator for 48/96 hours; if the incubation is extended to 96 hours, the culture medium is changed with a fresh one;
- at the end of the incubation, the sealed envelope is opened and the collagen structure, with the surface thereof invaded by autologous skin cells under growing, is washed with sterile saline so as to be ready for the implantation on the bed of the patient's wound.

## Claims

1. Disposable medical device for medication of skin lesions of a patient, comprising a sealed and sterilized external envelope (1) with an internal chamber or tray (2), a support (3) for skin cells housed inside said chamber or tray (2) and a first flexible tube (4) extending outwards from said chamber or tray (2) and through a corresponding part of the external envelope (1) and ending with an openable and hermetically re-closable sealed connector (5), which first flexible tube (4) is usable to introduce skin cells into the chamber or tray (2), **characterized in that** said support (3) is a flat, uniform, non-bored collagen sheet (3) suitable for adhesion of a suspension of epithelial or dermal-epithelial cells, said suspension consisting of crushed, shredded and homogenized skin fragments of the same patient, there being provided a second flexible tube (9) provided with a sterile filter (10), permeable to gas and water vapor but impermeable to liquids, which second flexible tube (9) extends freely outwards from said envelope (1) to create within said chamber or tray (2) an atmosphere suitable for growing the cells on said collagen sheet (3).

2. Method for preparing and using the disposable medical device according to claim 1, the method comprising the following steps:
- a small aliquot, of about 2 cm², of skin fragments of the same patient is crushed, shredded and homogenized and suspended in a small amount of a sterile saline to form a cell suspension;
- the cell suspension is distributed on the surface of the collagen sheet (3) of the device through an openable and hermetically reclosable opening of the sealed envelope (1);
- a selected culture medium, which is specific for growing fibroblasts and/or keratinocytes, is loaded inside the sealed envelope (1);
- the device is placed inside an incubator for 48/96 hours, the culture medium being changed with a fresh one if the incubation is extended to 96 hours;
- at the end of the incubation, the sealed envelope (1) is opened and the collagen sheet (3), with the surface thereof invaded by autologous skin cells under growing, is washed with sterile saline so as to be ready for the implantation on the bed of the patient's lesion.

3. Method according to claim 2, wherein:
- a bacteriostatic and bactericide solution is added if the operator is not sure on maintaining sterile conditions.

## Patentansprüche

1. Medizinische Einwegvorrichtung zur Medikation von Hautläsionen eines Patienten, aufweisend eine versiegelte und sterilisierte äußere Hülle (1) mit einer inneren Kammer oder Schale (2), einen im Inneren der Kammer oder Schale (2) aufgenommenen Träger (3) für Hautzellen, und einen ersten flexiblen Schlauch (4), der sich von der Kammer oder Schale (2) nach außen und durch einen entsprechenden Bereich der äußeren Hülle (1) erstreckt und in einem öffenbaren und hermetisch wiederverschließbaren, abgedichteten Verbinder (5) endet, wobei der erste flexible Schlauch (4) dazu verwendbar ist, Hautzellen in die Kammer oder Schale (2) einzubringen,
**dadurch gekennzeichnet, dass** der Träger (3) eine flache gleichmäßige, bohrungsfreie Kollagenfolie (3) ist, die für die Adhäsion einer Suspension von Epithel- oder Hautepithelzellen geeignet ist, wobei die Suspension aus zerstoßenen, zerkleinerten und homogenisierten Hautfragmenten des gleichen Patienten besteht, wobei ein zweiter flexibler Schlauch (9) vorhanden ist, der mit einem sterilen Filter (10) versehen ist, das für Gas und Wasserdampf durchlässig, aber für Flüssigkeiten undurchlässig ist, wobei sich der zweite flexible Schlauch (9) von der Hülle (1) frei nach außen erstreckt, um in der Kammer oder Schale (2) eine Atmosphäre zu schaffen, die für das Wachstum der Zellen auf der Kollagenfolie (3) geeignet ist.

2. Verfahren zum Bereitstellen und Verwenden der medizinischen Einwegvorrichtung nach Anspruch 1,
wobei das Verfahren folgende Schritte aufweist:
- ein kleines Aliquot, von etwa 2 cm², von Hautfragmenten des gleichen Patienten wird zerstoßen, zerkleinert und homogenisiert und in einer geringen Menge einer sterilen Kochsalzlösung suspendiert, um eine Zellsuspension zu bilden;
- die Zellsuspension wird durch eine öffenbare und hermetisch wiederverschließbare Öffnung der versiegelten Hülle (1) auf der Oberfläche der Kollagenfolie (3) der Vorrichtung verteilt;
- ein ausgewähltes Kulturmedium, das für das Wachstum von Fibroblasten und/oder Keratinozyten spezifisch ist, wird in die versiegelte Hülle (1) eingebracht;
- die Vorrichtung wird für 48/96 Stunden in einen Inkubator gesetzt, wobei das Kulturmedium durch ein frisches ersetzt wird, wenn die Inkubation auf 96 Stunden verlängert wird;
- am Ende der Inkubation wird die versiegelte Hülle (1) geöffnet und die Kollagenfolie (3), deren Oberfläche von autologen Hautzellen im Wachstum besiedelt ist, wird mit steriler Kochsalzlösung gewaschen, um für die Implantation auf das Wundbett des Patienten bereit zu sein.

3. Verfahren nach Anspruch 2,
- wobei eine bakteriostatische und bakterizide Lösung hinzugefügt wird, wenn der Bediener hinsichtlich der Aufrechterhaltung von sterilen Bedingungen nicht sicher ist.

## Revendications

1. Dispositif médical à usage unique pour le traitement de lésions cutanées d'un patient, comprenant une enveloppe externe étanche et stérilisée (1) avec une chambre ou plateau interne (2), un support (3) pour des cellules cutanées logé à l'intérieur de ladite chambre ou plateau (2) et un premier tube flexible (4) s'étendant vers l'extérieur depuis ladite chambre ou plateau (2) et à travers une partie correspondante de l'enveloppe externe (1) et se terminant par un connecteur étanche (5) qui peut être ouvert et qui peut être refermé hermétiquement, lequel premier tube flexible (4) est utilisable pour introduire des cellules cutanées dans la chambre ou plateau (2), **caractérisé en ce que** ledit support (3) est une feuille de collagène (3) plane, uniforme, non perforée appropriée à l'adhésion d'une suspension de cellules épithéliales ou dermo-épithéliales, ladite suspension consistant en fragments de peau broyés, déchiquetés et homogénéisés du même patient, un second tube flexible (9) muni d'un filtre stérile (10), perméable aux gaz et à la vapeur d'eau mais imperméable aux liquides étant prévu, lequel second tube flexible (9) s'étend librement vers l'extérieur depuis ladite enveloppe (1) pour créer à l'intérieur de ladite chambre ou plateau (2) une atmosphère appropriée à la croissance des cellules sur ladite feuille de collagène (3).

2. Procédé pour préparer et utiliser le dispositif médical à usage unique selon la revendication 1, le procédé comprenant les étapes suivantes:
- une petite aliquote, d'environ 2 cm², de fragments de peau du même patient est broyée, déchiquetée et homogénéisée et mise en suspension dans une petite quantité d'un sérum physiologique stérile pour former une suspension cellulaire;
- la suspension cellulaire est répartie sur la surface de la feuille de collagène (3) du dispositif par une ouverture qui peut être ouverte et qui peut être refermée hermétiquement de l'enveloppe étanche (1);
- un milieu de culture sélectionné, qui est spécifique pour faire croître des fibroblastes et/ou des kératinocytes, est chargé à l'intérieur de l'enveloppe étanche (1);
- le dispositif est placé à l'intérieur d'un incubateur pendant 48/96 heures, le milieu de culture étant remplacé par un milieu frais si l'incubation est prolongée à 96 heures;
- à la fin de l'incubation, l'enveloppe étanche (1) est ouverte et la feuille de collagène (3), dont la surface est envahie par des cellules cutanées autologues en croissance, est lavée avec du sérum physiologique stérile afin d'être prête pour l'implantation sur le lit de la lésion du patient.

3. Procédé selon la revendication 2, dans lequel:
- une solution bactériostatique et bactéricide est ajoutée si l'opérateur n'est pas sûr du maintien de conditions stériles.
